# DEMANDE DE BREVET EUROPEEN

(11) **EP 0 888 782 A1**
(43) Date de publication de la demande: **07.01.1999**
(21) Numéro de dépôt: 98401599.0
(22) Date de dépôt: 26.06.1998
(51) Int. Cl.: A61L 9/20, A61L 9/12, B01D 53/86, D06M 13/00, F24F 3/16

(54) **Procédé et dispositif pour le traitement anti-odeur de l'air**

(30) Priorité: 04.07.1997 FR 9708522
(71) Demandeur: ELF ATOCHEM S.A., 92800 Puteaux, Hauts-de-Seine (FR)
(72) Inventeur: Caupin, Henri-Jean, 78000 Versailles (FR); Dussaud, Joseph, 38200 Vienne (FR)
(74) Mandataire: Haicour, Philippe

(57) **Abrégé**

On combine dans un système de renouvellement ou de brassage de l'atmosphère l'action photocatalytique du dioxyde de titane et l'activité anti-odeur de l'acide undécylénique ou de ses dérivés. L'invention consiste à interposer sur le trajet de l'air un filtre imprégné de ces deux substances et exposé à une source naturelle ou artificielle de rayonnement ultraviolet.

## Description

La présente invention a trait au traitement des nuisances olfactives et à l'assainissement de l'air, aussi bien dans les milieux ouverts que dans les enceintes fermées.

La désodorisation des atmosphères est un problème complexe encore mal résolu. La méthode la plus efficace consiste encore à procéder à des renouvellements massifs d'air dans des enceintes fermés, avec des taux horaires courants de 3 à 5 fois par heure dans les locaux à forte concentration humaine, situation aggravée par un tabagisme incontrôlable. On en connaît les incidences pour les individus, les moins graves étant l'imprégnation des vêtements, de la peau, des cheveux, les plus préoccupants étant celles de santé publique, les plus immédiatement chiffrables étant les dépenses en énergie de renouvellement de l'atmosphère, tant en chaleur en hiver qu'en refroidissement en été.

Il est connu (voir P. Pichat, actes du Congrès Eurodeur 97, 25-26 juin 1997, Paris) que le dioxyde de titane (TiO₂) développe une activité photocatalytique sous l'effet du rayonnement ultraviolet, qui en présence d'air génère des radicaux libres " O* ". Ces radicaux libres attaquent les composés chimiques adsorbés à la surface du TiO2, et par une succession de réactions chimiques impliquant l'oxygène de l'air, les dégradent le carbone organique jusqu'au stade ultime de CO2. Cette activité à été mise à profit pour la destruction des nuisances olfactives dans divers dispositifs d'assainissement de l'air. Cependant, cette action photocatalytique du TiO2 est tributaire d'une cinétique globale lente et les odeurs que l'on souhaite détruire ne disparaissent qu'avec un certain retard par rapport au moment du démarrage du traitement.

On sait aussi que les molécules undécyléniques (acide undécylénique, ses sels et esters, alcool undécylénique, aldéhyde undécylénique et leurs dérivés chimiques immédiats), - ci-après "dérivés undécyléniques" - développent une activité anti-odeur puissante à faibles doses sur des substances organiques perçues comme très désagréables, par exemple les produits de décomposition des déjections animales (voir FR-A-2655878). On a appliqué cette propriété à la désodorisation des cartons et papiers (voir FR-A-2742663). Toutefois, des problèmes de persistance de cette activité se posent avec les filtres de papier simplement imprégnés de dérivés undécyléniques, les gaz nauséabonds et chargés qui les lèchent ou qui les traversent y abandonnant aussi des corps gras dont l'accumulation en colmate la porosité et inhibe les molécules de dérivés undécyléniques en les noyant dans un magna graisseux.

On vient maintenant de trouver qu'il est possible de remédier à ces inconvénients en réalisant des filtres en papier chargés ou imprégnés à la fois de TiO2 et d'un dérivé undécylénique, et en équipant avec ces filtres des systèmes de ventilation dans lesquels ils sont en outre irradiés par une source de rayons ultraviolets lorsqu'ils ne sont pas euxmêmes exposés à une source naturelle d'UV.

Pour réaliser les filtres qui sont les moyens de l'invention et qui font eux-mêmes partie intégrante de l'invention, on fixe de 3 à 10 g/m² de TiO2 et de 3 à 20 g/m² de dérivé undécylénique sur des surfaces de supports poreux, par exemple des feuilles de papier non encollé à forte porosité, des plaques de papier gaufrés, ou un non tissé à mailles larges.

La coopération du TiO2 et du dérivé undécylénique dans le fonctionnement des filtres de l'invention se révèle étonnamment efficace au plan de la qualité de l'air, et l'on observe en parallèle une augmentation très sensible de la durée de vie du filtre dont la salissure progressive semble bien n'être due, pour l'essentiel, qu'à la retenue des particules inorganiques. La bonne qualité de l'air traité est immédiate, dès la mise en route du système.

Le dosage des dérivés undécyléniques rémanents dans le filtre après une certaine durée de fonctionnement est un moyen de contrôle du taux de lessivage possible du produit dans le temps, et les concentrations maximales possibles des dérivés undécyléniques dans les atmosphères.

L'invention s'applique à toute forme de contrôle d'atmosphère confinée avec des cycles respiratoires, animaux ou humains. Elle trouvera une application intéressante dans le conditionnement des véhicules automobiles, la ventilation des compartiments de chemin de fer, notamment les compartiments fumeurs, des cellules d'avions, des salles de restaurants, dans les équipements de hottes de cuisines à recyclage interne, la présente liste n'étant nullement limitative.

### EXEMPLE:

La grille de ventilation d'un local d'une discothèque à plafond bas d'un volume d'environ 120 m³, à forte densité d'occupation de fumeurs, a été équipée d'une feuille de papier A4 imprégné de 5 g/m² de TiO₂ et 3 g/m² d'acide undécylénique et d'un dispositif d'irradiation U.V. de cette feuille. On a ainsi a assuré un protection contre la montée des odeurs pendant 7 soirées successives, et ce n'est qu'après ce délai qu'on a pu constater que la feuille de papier imprégné virait au noir. Pendant toute cette période, les utilisateurs de la discothèque ont pu constater que les odeurs de tabac ont disparu de l'atmosphère, à la grande différence de ce qui se passait avec l'équipement initial.

## Revendications

1. Procédé d'épuration de l'air qui consiste à placer dans un circuit de renouvellement d'air ou un appareil de ventilation, un filtre imprégné de TiO₂ et d'un dérivé undécylénique irradié par une source de rayonnement ultraviolet.

2. Procédé selon la revendication 1, dans lequel on utilise un filtre imprégné de TiO2 à raison de 3 à 10 g/m2 et d'un dérivé undécylénique, à raison 3 à 20 g/m².

3. Procédé selon les revendications 1 ou 2, dans lequel le filtre est constitué par une feuille de papier non encollé à forte porosité, une structure en papier gaufré, ou un non tissé à large mailles.

4. Filtre à air constitué par une feuille de papier non encollé à forte porosité, une structure en papier gaufré, ou un non tissé à large mailles et imprégné de TiO2 à raison de 3 à 10 g/m2 et d'un dérivé undécylénique, à raison 3 à 20 g/m².

5. Un dispositif de circulation et d'épuration d'air comprenant au moins un système de ventilation actionnant une colonne d'air, sur le trajet de laquelle se trouve interposé un filtre un filtre imprégné de TiO₂ et d'un dérivé undécylénique irradié par une source naturelle ou artificielle de rayonnement ultraviolet.
